# EUROPÄISCHE PATENTSCHRIFT

(11) **EP 2 717 711 B1**
(45) Veröffentlichungstag und Bekanntmachung des Hinweises auf die Patenterteilung: **05.10.2016**
(21) Anmeldenummer: 12725797.0
(22) Anmeldetag: 06.06.2012
(51) Int. Cl.: A23J 1/00, A23J 1/14, C07K 1/14

(54) **VERFAHREN ZUR GEWINNUNG VON PROTEINEN AUS EINEM NATIVEN STOFFGEMENGE**
METHOD FOR OBTAINING PROTEINS FROM A NATIVE SUBSTANCE MIXTURE
PROCÉDÉ D'EXTRACTION DE PROTÉINES À PARTIR D'UN MÉLANGE DE MATIÈRES NATIVES

(30) Priorität: 07.06.2011 DE 102011050905
(43) Veröffentlichungstag der Anmeldung: 16.04.2014
(73) Patentinhaber: GEA Mechanical Equipment GmbH, 59302 Oelde (DE)
(72) Erfinder: HRUSCHKA, Steffen, 59302 Oelde (DE); BOSZULAK, Wladislawa, 59302 Oelde (DE)
(74) Vertreter: Specht, Peter
(86) Internationale Anmeldenummer: PCT/EP2012/060675
(87) Internationale Veröffentlichungsnummer: WO 2012/168288

(56) Entgegenhaltungen:
- DE-C- 810 102
- DE-C- 865 044
- GB-A- 573 721
- US-A- 3 798 126
- ABAYOMI P ADEBIYI ET AL: "Isolation and characterization of protein fractions from deoiled rice bran", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, Bd. 228, Nr. 3, 27. August 2008 (2008-08-27), Seiten 391-401, XP019653077, ISSN: 1438-2385

## Beschreibung

Die vorliegende Erfindung betrifft Verfahren zur Gewinnung von Proteinen aus einem nativen Stoffgemenge.

Die DE 195 29 795 C2 offenbart ein Verfahren, welches die Gewinnung von Ölen, Fetten oder Wachsen ermöglicht. Dabei wird ein wässriger Brei in einer Zentrifuge in feste und flüssige Bestandteile aufgetrennt. Zu dem wässrigen Brei wird ein Anteil von 5-75 Gew.%, bezogen auf den Flüssiganteil des Breis, eines organischen Lösungsmittels zugegeben. Die DE 195 29 795 C2 setzt dabei bei der Aufgabe an, aus dem wässrigen Brei eine blanke Ölphase, eine Wasserphase und eine von Öl befreite Feststoffphase zu isolieren. Dieses Verfahren hat sich grundsätzlich für die Gewinnung von Ölen, Wachsen und Fetten bewährt.

Die DE 810 102 C offenbart ein Verfahren in welchem ein entfetteter Pflanzensamen mit 70-90%igem Alkohol behandelt wird.

Die US2005/003061 A1 beschreibt ein Verfahren in welchem stufenweise Reiskleie aufgearbeitet wird. Dabei werden in einem ersten Verfahrensschritt Feststoffe aus einer wäßrigen Reiskleielösung abgetrennt und zu Pellets verpresst. In einem weiteren Verfahrensschritt werden im alkalischen Milieu Proteine abgetrennt. Schließlich werden im sauren Milieu unter Alkoholzugabe nochmals Proteine abgetrennt.

Bekannte Verfahren zur Proteinherstellung sind die Proteinisolatherstellung bei alkalischem pH-Wert oder die Proteinkonzentratherstellung bei saurem pH-Wert, welche vorzugsweise bei aus Hexan extrahierten Schrot angewandt werden, welche jedoch in Verbindung mit dem Verfahren der DE 195 29 795 C2 auf ein Protein-Lezithin-Gemisch ohne energieintensiven Trocknungsschritt nicht anwendbar sind.

Vor diesem Hintergrund ist es die Aufgabe der Erfindung eine Proteinphase hoher Reinheit zu gewinnen.

Die Erfindung löst diese Aufgabe durch die Merkmale des Anspruchs 1.

Erfindungsgemäß weist ein Verfahren zur Gewinnung von Proteinen aus nativen Stoffgemengen, insbesondere aus Leguminosen- oder Rapsschrot, bei dem das Stoffgemenge zunächst fein zerkleinert und ggf. (wenn nicht flüssig genug) durch Zugabe einer Flüssigkeit zu einem fließfähigen Brei verarbeitet wird, zumindest die folgenden Schritte auf:
i) Einstellen eines pH-Wertes des Breis in einen alkalischen Bereich, also auf einen pH-Wert größer als 7.0;
ii) Zugeben mindestens eines wasserlöslichen organischen Lösemittels im Anschluss an das Einstellen des alkalischen pH-Wertbereichs in den alkalischen Bereich; und
iii) Abtrennen einer Proteinphase aus dem Brei.

Die Reihenfolge dieser Schritte einzuhalten, ist besonders vorteilhaft.

Dabei erfolgt, anders als in der DE 195 29 795 C2, vor der Zugabe des wasserlöslichen organischen Lösemittels, ein Einstellen eines pH-Wertes des Breis in einen alkalischen Bereich. Dadurch wird die Löslichkeit der Proteine im wässrigen Medium erhöht, sie werden teilweise angelöst und liegen, sofern sie nicht vollständig aufgelöst sind, zumindest feinverteilt und voluminös in der Lösung vor und nicht in kompakter Form wie die übrigen Feststoffe. Eine vollständige Löslichkeit der Proteine ist durch das Vorliegen eines Protein-Lezithin-Gemisches gestört. In Anschluss an die Einstellung des pH-Wertes erfolgt die Zugabe des organischen wasserlöslichen Lösemittels, wodurch u.a. Öl aus der angelösten Proteinsuspension verdrängt wird.

Das erfindungsgemäße Verfahren ermöglicht es somit, Proteine mit hoher Reinheit zu gewinnen, da u.a. durch die Erhöhung der Löslichkeit der Proteine offenbar auch Bindungen beispielsweise zu Verunreinigungen aus Zellulose bzw. Schalen und dergleichen gelockert werden.

Das Verfahren kann zur Proteingewinnung genutzt werden. Darüber hinaus kann es zudem besonders vorteilhaft mit einer Gewinnung von Öl aus dem Stoffgemenge kombiniert werden, welches durch die Zugabe des Lösemittels in Schritt ii als separate Phase abgetrennt werden kann.

Weitere vorteilhafte Ausgestaltungen der Erfindung sind Gegenstand der Unteransprüche.

Bevorzugt erfolgt nach dem Schritt ii, also dem Anlösen der Proteine, ein Abtrennen von Feststoffen bzw. ungelöstem Sediment vor dem eigentlichen Abtrennen der Proteinphase und optional der Ölphase in einem getrennten Schritt.

Der pH-Wert in Schritt i ist vorzugsweise gleich oder größer pH=9. Durch die in Schritt i vorgenommene pH-Wert Verschiebung in den alkalischen Bereich wird ein besonders gutes Lösen oder Anlösen von Proteinen in der wässrigen Lösung erreicht. Dadurch kann eine bessere Trennung der Proteinphase von den restlichen Feststoffen erfolgen. Besonders günstige Bedingungen zum Anlösen der Proteine werden bei einem pH-Wert von größer als pH=9, und insbesondere bei einem pH-Wert von pH=10±0.5, erreicht.

Als wasserlösliches organisches Lösemittel aus Schritt ii bietet sich erfindungsgemäß ein kurzkettiger aliphatischer Alkohol an. Dies betrifft in erster Linie leicht verfügbare Alkohole, wie Methanol, Ethanol oder Propanol, welche in großem Umfang verfügbar sind.

Da die Zugabe des Lösungsmittel mit einer Senkung der Löslichkeit der Proteine einhergeht, ist es von Vorteil, wenn der Gehalt an wasserlöslichem organischen Lösemittel in dem Brei nach dem Zugeben des wasserlöslichen alkoholischen Lösemittels in Schritt ii weniger als 45 Vol.%, vorzugsweise ≤15 Vol.% beträgt. Durch eine erhöhte Konzentration oberhalb von 45 Vol.% an alkoholischen Lösemittel wird das ggf. abzutrennende Öl in eine Zwischenphase zwischen der Proteinphase und der wässrigen Phase verdrängt. Dies erschwert die Abtrennung der Ölphase und führt zu weniger guten Ergebnissen als unterhalb von 45 Vol.%. Die Proteine bleiben kompakt und vermischen sich mit der Feststoffphase.

Besonders geeignet für das Abtrennen der Feststoffphase ist die Trennung in einem Zentrifugalfeld. Das Abtrennen der Feststoffphase kann vorzugsweise mittels eines Klärdekanters erfolgen.

Nach dem Entfernen der Feststoffe liegt ein Gemisch aus wässriger alkoholischer Lösung und Proteinen in einer im Wesentlichen wässrigen Form und eine Ölphase vor. Es ist nunmehr von Interesse, die wertvollen Inhaltsstoffe, also die Proteinphase und die Ölphase zu isolieren. Das Isolieren zumindest der Proteinphase in Schritt iii erfolgt vorzugsweise mit dem Schritt iii-1, dem Fällen der Proteinphase durch Einstellen des pH-Wertes. Dadurch liegen in dem Gemisch eine Feststoffphase und einer oder zwei Flüssigphasen vor, welche sich in einem anschließenden Schritt iii-2 im Zentrifugalfeld in eine Ölphase, eine Proteinphase und eine alkoholisch-wässrige Phase trennen lassen. Dies kann vorzugsweise mittels eines Dreiphasen-Separators erfolgen.

Das Fällen der Proteinphase erfolgt vorzugsweise durch Senkung des pH-Wertes auf den isoelektrischen Punkt. Dabei können sich u.a. auch einzelne ausgefällte Proteine zusammenballen, wodurch diese noch besser von den flüssigen Phasen getrennt werden können.

Zur Verbesserung der Reinheit der Proteinphase kann diese nach dem Isolieren durch Einstellen des pH-Wertes in Schritt iv gewaschen werden.

Das gewonnene Protein ist "nativ" und weitgehend polyphenolfrei.

Die Erfindung wird nachfolgend anhand eines Ausführungsbeispiels mit Bezug auf die beigefügten Zeichnungen erläutert. Sie zeigen:

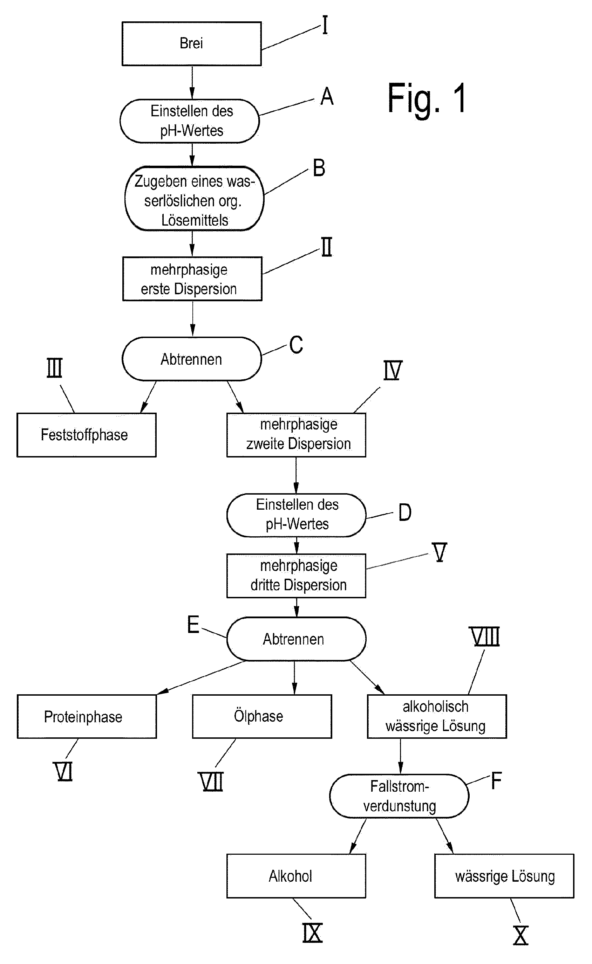

- Figur 1: ein Diagramm eines beispielhaften Verfahrensablaufes

Im Folgenden wird das erfindungsgemäße Verfahren anhand der in Fig.1 dargestellten konkreten Schrittfolge näher ausgeführt.

Als Ausgangsstoff wird ein natives organisches Stoffgemenge, vorzugsweise Leguminosen, Rapssaat oder Mikroorganismen, eingesetzt. Dieses Stoffgemenge wird erfindungsgemäß zunächst zerkleinert und durch Zugabe von Wasser zu einem fließfähigen Brei umgesetzt.

Dieser Brei I kann beispielsweise aus einem Presskuchen aus der Ölgewinnung hergestellt sein, welcher zusammen mit Wasser zu dem Brei I suspendiert wurde. Besonders wird der Brei I aus der Rapssaat oder Sojabohnen gewonnen. Der Brei I enthält zusätzlich zu Ölbestandteilen auch Proteine. Darüber hinaus kann der Brei I auch Lezithin, Polyphenole und Feststoffbestandteile, wie Schalen, enthalten, deren Gehalt sowohl in der zu gewinnenden Ölphase als auch in dem zu gewinnenden Proteinanteil möglichst gering sein sollen.

In einem ersten Verfahrensschritt A wird zu dem pH-Wert des Breis I beispielsweise durch Zugabe von Natiumhydroxidlösung in den alkalischen Bereich verschoben. Dadurch wird die Proteinlöslichkeit heraufgesetzt und die Proteine werden weitestgehend in Lösung gebracht. Ein geringer Anteil an Proteinen kann allerdings nach wie vor noch ungelöst allerdings feinverteilt im wässrigen Brei vorliegen, da die Löslichkeit der Proteine durch den Anteil an Öl und Lezithin im Stoffgemisch eingeschränkt ist. Der pH-Wert der Dispersion ist nach dem Schritt i erfindungsgemäß größer als pH=9, besonders bevorzugt ist der pH-Wert der Dispersion bei pH=10.

In einem zweiten Verfahrensschritt B wird die alkalische Dispersion anschließend erfindungsgemäß mit einem kurzkettigen aliphatischen Alkohol versetzt. Dieser Alkohol ist ausgewählt aus einer Gruppe von Alkoholen bestehend aus Methanol, Ethanol und/oder Propanol. Durch die Zugabe des Alkohols findet eine Verschiebung des Löslichkeitsgleichgewichtes statt. Es erfolgt eine sogenannte Verdrängungsextraktion, bei der das Öl aus der zerkleinerten nativen Stoffmatrix durch die Zugabe von Alkohol verdrängt wird.

Dabei trennt sich die alkoholisch-wässrige alkalische Dispersion II in insgesamt vier Phasen auf, eine Ölphase, eine Alkoholphase, eine Proteinphase und eine Feststoffphase aus Schalen und anderen Feststoffen. Das Volumen an zugegebenem Alkohol in Schritt ii ist dabei vorzugsweise so zu wählen, dass der Alkoholgehalt der wässrigen Dispersion nach Schritt ii von weniger als 45 Vol. % beträgt. Dabei hat sich ein Alkoholgehalt von ≤ 15 Vol. % als besonders günstig erwiesen um eine Phasentrennung zwischen der Proteinphase und der Schalenphase mit sehr reinen Einzelphasen zu erhalten. Besonders bevorzugt werden dabei auch die Bindungen zu anderen Verbindungen, so z.B. von Verunreinigungen aus Zellulose, beispielsweise aus Schalen, derart geschwächt, so dass im Zentrifugalfeld eine Trennung der alkoholisch-wässrigen alkalischen ersten Dispersion II mit den bereits zuvor beschriebenen mehreren Phasen erfolgt.

In Verfahrensschritt C erfolgt im Anschluss an die Alkoholzugabe eine erste Abtrennung, wobei die Feststoffphase aus Schalen und weiteren Bestandteilen aus der mehrphasigen ersten Dispersion II entfernt wird. Dieser Verfahrensschritt C erfolgt vor der eigentlichen Proteintrennung und ermöglicht das Entfernen von unerwünschten Feststoffen. Diese Abtrennung von Feststoffen erfolgt bevorzugt als zentrifugale Abtrennung in einem Klärdekanter.

Nach der Abtrennung erhält man eine reine Feststoff-Fraktion II und eine mehrphasige zweite Dispersion IV aus zumindest einer oberen Ölphase, einer alkoholisch-wässrigen mittleren Phase und einer unteren suspendierten Proteinphase.

In einem sich anschließenden Verfahrensschritt D, bzw. einem Schritt iv, erfolgt eine Fällung der Proteine durch Einstellung des pH-Wertes in dem Bereich des isoelektrischen Punkts der Proteinphase, wobei mehrphasige dritte Dispersion V ausgebildet wird, mit einer Protein-Feststoffphase und zwei Flüssigphasen, einer Öl- und einer alkoholisch-wäßrigen Phase.

Nach dem Ausfällen der Proteine erfolgt in einem Verfahrensschritt E, bzw. einem Schritt v, eine zweite Abtrennung. Diesmal werden allerdings die Proteinphase, die Ölphase und die alkoholisch-wässrige Phase voneinander getrennt. Dies erfolgt besonders bevorzugt durch eine zentrifugale Abtrennung.

Nach dem Verfahrensschritt E erhält man eine Proteinphase VI, eine Ölphase VII und eine alkoholisch wässrige Phase VIII, in einem oder mehreren Schritten.

In einem weiteren optionalen Verfahrensschritt F kann der Alkohol IX aus der alkoholisch-wässrigen Phase VII durch Fallstromverdampfung zurückgewonnen werden. Als Rückstand aus dem Prozess bleibt somit eine im Wesentlichen wässrige Lösung X zurück.

Bei der Untersuchung der gewonnenen Produkte (Öl und Proteine) hat sich gezeigt, dass ein verbessertes Trennverhalten und damit verbunden auch eine noch höhere Reinheit der Produkte und hier insbesondere der gewonnenen Proteine erreicht werden konnte. Störende Verunreinigungen, wie z.B. Polyphenole und Lezithin reicherten sich in besonders hohem Maße in der alkoholisch-wässrigen Phase VIII an, wobei Polyphenole nicht mehr oder nur noch in verschwindend geringen Anteilen in der abgetrennten und ggf. gewaschenen Proteinphase vorgefunden wurden.

Es hat sich zudem gezeigt, dass eine gleichzeitige Zugabe von Lauge und Alkohol und auch eine Umkehrung der Schritte i und ii, wie sie im Anspruch 1 beschrieben werden, nicht dazu führt, dass ein ausreichendes Anlösen der Proteine erfolgt und damit ein Isolieren einer von Verunreinigungen befreiten Proteinphase nur unzureichend erfolgt und bei geringerer Proteinausbeute erfolgt.

Konkret äußert sich die verbesserte Trennung zwischen Proteinphase und Feststoffen nach Zugabe einer Lauge in Schritt i oder Verfahrensschritt A derart, dass sich eine erste Dispersion II mit mehrere Phasen wie folgt ausbilden:

| | |
|---|---|
| Oberste Phase: | Öl (gelbe Farbe) |
| Zweite Phase: | Wässrige Alkoholphase (trüb bräunlich) |
| Dritte Phase: | Proteinphase mit angelösten Proteinen (weiß-gelbe Phase) |
| Bodensatz: | Feststoffphase aus Schalen und dergl. (schwarz-grüne Phase) |

Dabei waren bei der Variation der Schrittabfolge der Schritte i und ii bzw. der Verfahrensschritte A und B unterschiedliche Reinheiten der Feststoffphase zu beobachten. So war die Feststoffphase bei der Schrittfolge nach dem erfindungsgemäßen Verfahren grünschwarz und wiesen wenig weiße Zwischenräume von Proteinen, also nur eine geringe Marmorierung, auf. Anders verhielt es sich bei umgekehrter Schrittabfolge - Schritt ii vor Schritt i - und bei gleichzeitiger Zugabe von Alkohol und Lauge. Hier war eine intensivere Marmorierung und damit eine unerwünschte Vermischung beider Phasen, also der Protein- und der Feststoffphase, zu beobachten.

Nach der zentrifugalen Abtrennung der Feststoffe III nach Verfahrensschritt C und der Senkung des pH-Wertes auf den isoelektrischen Punkt zur Fällung der Proteine nach Schritt iv bzw. Verfahrensschritt D liegen folgende Phasen in der mehrphasigen dritten Dispersion V vor:

| | |
|---|---|
| Oberste Phase: | Öl (gelbe Farbe) |
| Zweite Phase: | Wässrige Alkoholphase (trüb bräunlich) |
| Dritte Phase: | Proteinphase mit ausgefällten Proteinen (weiß-gelbe Phase) |

Die Proteine können nun genau wie das Öl vorzugsweise in eine anschließenden zentrifugalen flüssig-flüssig-fest Trennverfahren in Verfahrensschritt E isoliert werden. Dabei war auffällig, dass ein Großteil an Lezithin und Polyphenolen, welche bislang verstärkt in der Proteinphase gefunden wurden, nunmehr in der alkoholisch-wässrigen Phase verstärkt zu finden sind, während die Proteinphase eine höhere Reinheit aufweist.

Weiterhin kann bei dem Verfahren auf den Einsatz von Schutzgas vorteilhaft verzichtet werden.

Anstelle der beschriebenen Ölphase VI können beispielsweise auch Fette oder Wachse auf gleiche Art und Weise aus dem Brei abgetrennt werden. Beispielhaft wurde mit dem erfindungsgemäßen Verfahren eine Rapsprobe verarbeitet, um Proteine zu gewinnen.

Ein entsprechender Rapspresskuchen (100g) bestand gemäß Analyse zu 90 Gew% aus Trockensubstanz, wovon 31,77 % Proteine waren, 18,31 Prozent Öle, 1,71 Gew% PP (Polyphenol) und ca. 10 Gew% Wasser.

Der zu verarbeitende Raps (100 g) wurde zunächst mit dem Scherkopfmischer unter Zugabe von 415 g destilliertem Wasser fein zerkleinert und zu einem fließfähigen Brei verarbeitet, wobei sodann durch Zugabe von 10%iger Lauge ein Einstellen des pH-Wertes des Breis von 10 in einen alkalischen Bereich (Verfahrensschritt A) erfolgte.

Anschließend wurde der Brei bei 30 min schonen gemischt.

Sodann wurden diesem Brei 78,5g Alkohol als wasserlösliches organisches Lösemittels im Anschluss an das Einstellen des pH-Wertes des Breis zugegeben.

Sodann wurde der Brei für 2 min bei 40°C zentrifugiert und aus den Schleuderfraktionen wurde eine Proteinphase abgetrennt, die sich im Becherglas als untere Schicht oberhalb der sauber abgetrennten Schalen mit einem Volumenanteil von 40% abgesetzt hatte.

Es konnte so ein Eiweiß-Anteil von 18,28 g (68,3 %) abgetrennt werden, der weitestgehend polyphenolfrei war. Der Eiweißanteil war auch ansonsten sehr rein, insbesondere ersichtlich frei von Schalenanteil und frei von sonstigen sichtbaren Verunreinigungen. Hier zeigt sich ein wesentlicher Vorteil der Einhaltung der Schritte pH-Wert-Verschiebung, Zugabe des wasserlöslichen organischen Lösemittels und dann direkt oder nach weiteren Zwischenschritten c) Abtrennen der Proteinphase, da die Proteinphase besonders rein ist.
- I: Brei
- II: mehrphasige erste Dispersion
- III: Feststoffphase
- IV: mehrphasige zweite Dispersion
- V: mehrphasige dritte Dispersion
- VI: Proteinphase
- VII: Ölphase
- VIII: alkoholisch wässrige Lösung
- IX: Alkohol
- X: wässrige Lösung

- Verfahrensschritt A: Einstellen des pH-Wertes
- Verfahrensschritt B: Zugeben eines wasserlöslichen org. Lösemittels
- Verfahrensschritt C: Abtrennen
- Verfahrensschritt D: Einstellen des pH-Wertes
- Verfahrensschritt E: Abtrennen
- Verfahrensschritt F: Fallstromverdunstung

## Patentansprüche

1. Verfahren zur Gewinnung von Proteinen aus nativen Stoffgemengen aus einem Presskuchen aus Rapssaat aus der Ölgewinnung, bei dem ein natives Stoffgemenge aus dem Presskuchen der Ölgewinnung aus der Rapssaat zunächst fein zerkleinert und durch Zugabe der Flüssigkeit Wasser zu einem fließfähigen Brei (I) verarbeitet wird, der zusätzlich zu Ölbestandteilen auch Proteine, Lezithin, Polyphenole und als Feststoffbestandteile insbesondere Schalen enthält,
**gekennzeichnet durch** die folgenden Schritte:
i Einstellen des pH-Wertes des Breis (I) in einen alkalischen Bereich , so dass der pH-Wert größer als pH=9 ist,
ii Zugeben mindestens eines wasserlöslichen organischen Lösemittels in Form eines kurzkettioen aliphatischen Alkohols, ausgesucht aus einer Gruppe von Alkoholen bestehend aus Methanol, Ethanol und/oder Propanol, im Anschluss an das Einstellen des pH-Wertes des Breis, derart, dass **durch** die Zugabe des Alkohols eine Verschiebung des Löslichkeitsgleichgewichtes stattfindet und eine Verdrängungsextraktion erfolgt, wobei sich die entstehende alkoholisch-wässrige alkalische Dispersion in insgesamt folgende vier Phasen aufteilt: eine Ölphase, eine Alkoholphase, eine Proteinphase und eine Feststoffphase aus Schalen und anderen Feststoffen, wobei das Volumen an zugegebenem Alkohol in Schritt ii so gewählt ist, dass der Alkoholgehalt der wässrigen Dispersion nach Schritt ii weniger als 45 Vol. % beträgt, und
iii Abtrennen einer Proteinphase (VI) aus dem Brei im Anschluss an das Zugeben des wasserlöslichen Lösemittels gemäß. Schritt ii, wobei vor dem Abtrennen der Proteinphase ein Abtrennen der Feststoffphase (III) aus dem Brei (I) erfolgt.

2. Verfahren nach Anspruch 1, **dadurch gekennzeichnet, dass** ein Abtrennen einer Ölphase (VII), eines Fettes oder eines Wachses aus dem Brei im Anschluss an Schritt ii erfolgt.

3. Verfahren nach Anspruch 1 oder 2, **dadurch gekennzeichnet, dass** die Abtrennung der Ölphase in einem oder mehreren Schritten erfolgt.

4. Verfahren nach Anspruch 3, **dadurch gekennzeichnet, dass** die Abtrennung in einem 3-Phasendekanter oder in zumindest zwei Schritten in 2-Phasendekantern erfolgt.

5. Verfahren nach einem der vorhergehenden Ansprüche, **dadurch gekennzeichnet, dass** der pH-Wert in Schritt i pH=10±0.5 ist.

6. Verfahren nach einem der vorhergehenden Ansprüche, **dadurch gekennzeichnet dass** das Einstellen des alkalischen pH-Wertes des Breis (I) durch Zugabe einer Lauge erfolgt.

7. Verfahren nach Anspruch 7, **dadurch gekennzeichnet, dass** die Lauge eine Natriumhydroxidlösung ist.

8. Verfahren nach einem der vorhergehenden Ansprüche, **dadurch gekennzeichnet, dass** das wasserlösliche organische Lösemittel aus Schritt ii ein linearer aliphatischer Alkohol ist.

9. Verfahren nach einem der vorhergehenden Ansprüchen, **dadurch gekennzeichnet, dass** der Gehalt an wasserlöslichem organischen Lösemittel in dem Brei (I) nach dem Zugeben des wasserlöslichen alkoholischen Lösemittels in Schritt ii weniger als 15. % beträgt.

10. Verfahren nach Anspruch 3, **dadurch gekennzeichnet, dass** das Abtrennen der Feststoffphase (III) in einem Zentrifugalfeld erfolgt.

11. Verfahren nach einem der vorhergehenden Ansprüche, **dadurch gekennzeichnet, dass** das Abtrennen der Feststoffphase (III) mittels eines Klärdekanters erfolgt.

12. Verfahren nach einem der vorhergehenden Ansprüche, **dadurch gekennzeichnet, dass** das Abtrennen zumindest der Proteinphase in Schritt iii mit folgenden Schritten erfolgt:
iv Fällen der Proteinphase (VI) durch Einstellen des pH-Wertes ; und
v Zentrifugales Trennen der Proteinphase (VI), einer alkoholisch-wässrigen Phase (VIII) und ggf. einer Ölphase (VII).

13. Verfahren nach Anspruch 13, **dadurch gekennzeichnet, dass** das Fällen der Proteinphase (VI) durch Senkung des pH-Wertes auf den isoelektrischen Punkt der Proteine erfolgt.

14. Verfahren nach einem der vorhergehenden Ansprüche, **dadurch gekennzeichnet, dass** die Proteinphase (VI) nach dem Isolieren in Schritt iii gewaschen wird.

15. Verfahren nach einem der vorhergehenden Ansprüche, **dadurch gekennzeichnet, dass** nach Schritt iii eine Rückgewinnung von Alkohol (IX) aus der alkoholisch-wässrigen Phase (VIII) erfolgt.

16. Verfahren nach Anspruch 15, **dadurch gekennzeichnet, dass** die Rückgewinnung von Alkohol (IX) durch eine Fallstromverdunstung erfolgt.

## Claims

1. Process for recovering proteins from natural product mixtures composed of a press cake from rapeseed from oil recovery, in which a natural product mixture composed of the press cake from oil recovery from rapeseed is firstly finely comminuted and processed by addition of the liquid water to form a flowable slurry (I) which in addition to oil constituents also contains proteins, lecithin, polyphenols and in particular husks as solid constituents, **characterized by** the following steps:
i setting of the pH of the slurry (I) in the alkaline range so that the pH is greater than pH = 9,
ii addition of at least one water-soluble organic solvent in the form of a short-chain aliphatic alcohol selected from a group of alcohols consisting of methanol, ethanol and/or propanol after setting of the pH of the slurry in such a way that the addition of the alcohol brings about a shift in the solubility equilibrium and a displacement extraction occurs, where the resulting alcoholic-aqueous alkaline dispersion separates overall into the following four phases: an oil phase, an alcohol phase, a protein phase and a solids phase composed of husks and other solids, where the volume of alcohol added in step ii is selected so that the alcohol content of the aqueous dispersion after step ii is less than 45% by volume, and
iii separation of a protein phase (VI) from the slurry after the addition of the water-soluble solvent in step ii, where a separation of the solids phase (III) from the slurry (I) is carried out before the protein phase is separated off.

2. Process according to Claim 1, **characterized in that** an oil phase (VII), a fat or a wax is separated off from the slurry after step ii.

3. Process according to Claim 1 or 2, **characterized in that** the isolation of the oil phase is carried out in one or more steps.

4. Process according to Claim 3, **characterized in that** the separation is carried out in a three-phase decanter or in at least two steps in two-phase decanters.

5. Process according to any of the preceding claims, **characterized in that** the pH in step i is pH = 10 ± 0.5.

6. Process according to any of the preceding claims, **characterized in that** the setting of the alkaline pH of the slurry (I) is effected by addition of an alkali.

7. Process according to Claim 7, **characterized in that** the alkali is a sodium hydroxide solution.

8. Process according to any of the preceding claims, **characterized in that** the water-soluble organic solvent in step ii is a linear aliphatic alcohol.

9. Process according to any of the preceding claims, **characterized in that** the content of water-soluble organic solvent in the slurry (I) after addition of the water-soluble alcohol solvent in step ii is less than 15%.

10. Process according to Claim 3, **characterized in that** the removal of the solid phase (III) is effected in a centrifugal field.

11. Process according to any of the preceding claims, **characterized in that** the removal of the solid phase (III) is effected by means of a clarifying decanter.

12. Process according to any of the preceding claims, **characterized in that** the isolation of at least the protein phase in step iii is carried out by means of the following steps:
iv precipitation of the protein phase (VI) by adjustment of the pH; and
v centrifugal separation of the protein phase (VI), an alcoholic-aqueous phase (VIII) and optionally an oil phase (VII).

13. Process according to Claim 13, **characterized in that** the precipitation of the protein phase (VI) is effected by lowering the pH to the isoelectric point of the proteins.

14. Process according to any of the preceding claims, **characterized in that** the protein phase (VI) is washed after isolation in step iii.

15. Process according to any of the preceding claims, **characterized in that** recovery of alcohol (IX) from the alcoholic-aqueous phase (VIII) is carried out after step iii.

16. Process according to Claim 15, **characterized in that** the recovery of alcohol (IX) is carried out by falling film evaporation.

## Revendications

1. Procédé pour l'extraction de protéines à partir de mélanges de matières natifs issus d'un tourteau de graines de colza résultant de l'extraction d'huile, dans lequel un mélange de matières natif issu du tourteau résultant de l'extraction d'huile à partir des graines de colza est d'abord broyé finement et transformé par addition d'eau en une bouillie fluide (I) qui contient, outre les composantes huileuses, des protéines, de la lécithine, des polyphénols et des solides, en particulier de la balle,
**caractérisé en ce qu'**il comprend les étapes suivantes :
i. ajustement du pH de la bouillie (I) dans une plage alcaline, de telle manière que le pH soit supérieur à pH = 9 ;
ii. ajout d'au moins un solvant organique hydrosoluble sous la forme d'un alcool aliphatique à chaîne courte, choisi parmi un groupe d'alcools comprenant le méthanol, l'éthanol et/ou le propanol, après l'ajustement du pH de la bouillie, de telle manière que l'ajout de l'alcool provoque un décalage de l'équilibre de solubilité et qu'il se produise une extraction par déplacement, la dispersion alcaline dans l'eau et l'alcool ainsi formée se divisant en quatre phases en tout : une phase huileuse pure, une phase alcoolique, une phase protéique et une phase solide composée de la balle et d'autres solides, le volume de l'alcool ajouté dans l'étape ii étant choisi de telle façon que la teneur en alcool de la dispersion aqueuse après l'étape ii soit inférieure à 45 % en volume, et
iii. séparation d'une phase protéique (VI) de la bouille après l'ajout du solvant hydrosoluble selon l'étape ii, la phase solide (III) étant séparée de la bouille (I) avant la séparation de la phase protéique.

2. Procédé selon la revendication 1, **caractérisé en ce qu'**une séparation d'une phase huileuse (VII), d'une graisse ou d'une cire de la bouillie est effectuée après l'étape ii.

3. Procédé selon la revendication 1 ou 2, **caractérisé en ce que** la séparation de la phase huileuse est effectuée en une ou plusieurs étapes.

4. Procédé selon la revendication 3, **caractérisé en ce que** la séparation est effectuée dans un décanteur à trois phases ou en au moins deux étapes dans des décanteurs à deux phases.

5. Procédé selon l'une des revendications précédentes, **caractérisé en ce que** le pH dans l'étape i est de pH = 10 ± 0,5.

6. Procédé selon l'une des revendications précédentes, **caractérisé en ce que** l'ajustement du pH alcalin de la bouillie (I) est effectué par ajout d'une base.

7. Procédé selon la revendication 7, **caractérisé en ce que** la base est une solution d'hydroxyde de sodium.

8. Procédé selon l'une des revendications précédentes, **caractérisé en ce que** le solvant organique hydrosoluble de l'étape ii est un alcool aliphatique à chaîne droite.

9. Procédé selon l'une des revendications précédentes, **caractérisé en ce que** la teneur en solvant organique hydrosoluble dans la bouillie (I) après l'ajout du solvant alcoolique hydrosoluble dans l'étape ii est inférieure à 15 %.

10. Procédé selon la revendication 3, **caractérisé en ce que** la séparation de la phase solide (III) est effectuée dans un champ centrifuge.

11. Procédé selon l'une des revendications précédentes, **caractérisé en ce que** la séparation de la phase solide (III) est effectuée au moyen d'un décanteur de clarification.

12. Procédé selon l'une des revendications précédentes, **caractérisé en ce que** la séparation de la phase protéique, au minimum, dans l'étape ii est effectuée selon les étapes suivantes :
iv précipitation de la phase protéique (VI) par ajustement du pH et
v séparation par centrifugation de la phase protéique (VI), d'une phase hydro-alcoolique (VIII) et éventuellement d'une phase huileuse (VII).

13. Procédé selon la revendication 13, **caractérisé en ce que** la précipitation de la phase protéique (VI) est effectuée en abaissant le pH jusqu'au point isoélectrique des protéines.

14. Procédé selon l'une des revendications précédentes, **caractérisé en ce que** la phase protéique (VI) est lavée après avoir été isolée dans l'étape iii.

15. Procédé selon l'une des revendications précédentes, **caractérisé en ce qu'**après l'étape iii, l'alcool (IX) est extrait de la phase hydro-alcoolique (VIII) et récupéré.

16. Procédé selon la revendication 15, **caractérisé en ce que** la récupération de l'alcool (IX) est effectuée par évaporation à descendage.
